# EUROPEAN PATENT SPECIFICATION

(11) **EP 2 831 157 B1**
(45) Date of publication and mention of the grant of the patent: **23.02.2022**
(21) Application number: 13769922.9
(22) Date of filing: 27.03.2013
(51) Int. Cl.: C08J 9/30, D21J 1/06, D21J 1/20, C09K 17/52, C09K 3/32, A01G 13/02, C05F 11/04, B29C 67/20, D21F 11/00, A01G 13/00, C05F 11/02

(54) **PEAT MOSS STRUCTURES**
TORFMOOSGRANULATSTRUKTUREN
STRUCTURES DE TOURBE À SPHAIGNES

(30) Priority: 28.03.2012 FI 20125353
(43) Date of publication of application: 04.02.2015
(73) Proprietor: Teknologian tutkimuskeskus VTT Oy, 02150 Espoo (FI)
(72) Inventor: IMMONEN, Kirsi, FI-33101 Tampere (FI); KINNUNEN, Karita, FI-02044 VTT (FI); LEHMONEN, Jani, FI-40101 Jyväskylä (FI); HJELT, Tuomo, FI-02044 VTT (FI); ERKKILÄ, Ari, FI-40101 Jyväskylä (FI)
(74) Representative: Boco IP Oy Ab
(86) International application number: PCT/FI2013/050342
(87) International publication number: WO 2013/144449

(56) References cited:
- EP-A1- 0 779 066
- EP-A1- 1 116 434
- EP-B1- 0 670 669
- WO-A1-96/02702
- GB-A- 519 724
- US-A- 4 676 871
- US-A- 4 777 763

## Description

### FIELD OF THE INVENTION

The invention relates to new applications of peat in composite structures. Particularly the invention relates to a method for the manufacture of composite structures comprising peat moss, and to a composite structure obtained by the method.

### BACKGROUND

Peat moss was used as an insulator material in buildings and structures in the northern countries for centuries, however, today said use is less popular. Many other fields of use for peat moss have been proposed and for example the use of peat moss, typically in combination with other fibrous materials for absorbent products, such as sanitary napkins, diapers, dressings and the like, as well as horticultural products, e.g., as mulch for growing plants, have been suggested.

US 4,215,692 relates to absorbent products made of screened peat moss, where fines and coarse material of harvested peat moss, primarily roots and branches are discarded and the remaining peat moss is bleached, formed into a board along with finely ground mechanical wood pulp and optionally long-fibered wood pulp, followed by drying. The absorbent may be treated to include a wetting agent in quantities less than 0.5 % by weight.

A low density peat moss board, suitable as absorbent, is disclosed in US 4,507,122, where carefully selected fractions of peat moss, together with mechanical pulp fines having Canadian Standard Freeness of 60 to 500 are mixed to an aqueous slurry, which is flowed onto a Fourdrinier wire where the slurry is dewatered and dried to form the board.

US 4,676,871 teaches absorbent boards and horticultural boards made of harvested peat moss having a degree of composition of H-1 value, said peat moss being individualized and dried, followed by air-laying the dispersed particles onto a foraminous substrate to produce the low density board, which is then calendered. A surfactant may be added prior to the individualization step. Long fibers, such as polyester, glass, polypropylene, nylon, polyacetate and rayon fibers may be added to the peat moss to impart additional strength.

US 4,473,440 relates to the manufacture of flexible absorbent boards comprising peat moss, useful for incorporating in diapers, sanitary napkins and certain industrial uses. Screened peat moss may be combined with other absorbent materials such as Kraft wood pulp and mechanical wood pulps. The obtained mixture is formed to a slurry, which is flowed to a Fourdrinier wire, dewatered and calendered. The slurry may also comprise coloring agents, wetting agents, adhesives, etc.

Boards and layered blankets comprising peat moss, for use in liquid and oil absorption, insulation, as ballistic products, and for acoustics and horticulture applications are offered commercially. Said boards and blankets may be manufactured as layered structures from peat moss and binders using heat treatment, as suggested in FI 20055272 and FI 20065675. In the heat treatment the binder is melted and upon cooling it binds the peat moss fibers together.

GB 519 724 A relates to a batch method for the manufacture of heat-insulation boards of peat, wherein the porous rigid structure for the board is obtained by using chemical agents.

EP 1 116 434 A1 discloses a foam containing natural fibers, granular material, binder, water and surfactant, which foam can be laid on the ground and allowed to dry to obtain a mulch mat to suppress weeds.

EP 0 670 669 B1 concerns sheet of mineral wool, where parts of plants are attached to the surface of the sheet by means of an organic polymer-containing adhesive, which is in the form of foam, and contains peat, fertilizers, adhesive dispersion or starch and additives.

WO 96/02702 A1 relates to a method for producing a foam-formed fiber or paper web from cellulose pulp for producing paper products such as soft paper, spun-lace material and the like.

EP 0 779 066 A1 discloses liquid absorbent sphagnum moss material containing an effective amount of cross-linked cellulosic fibers, suitable for disposable absorbent products such as sanitary napkins, incontinence diapers etc.

US 4 777 763 A relates to lofted-fiber plant growing boards containing glass fiber.

The prior art methods require the use of high amounts of water and they are energy consuming, or they need the use of polymeric binders, which requires heat treatment in the processing.

Technique relating to methods for producing foam-laid fiber webs is well known in the field of manufacture of non-woven and tissue products. Fiber web is formed from a dispersion of fibers in a foamed liquid. A pulp or fiber furnish is first prepared in a breaker, followed by dewatering, mixing with a foamable liquid containing a surfactant and water. The fibers are dispersed in the foam and the formed dispersion is deposited on a wire and the main portion of the liquid, which is essentially in the form of foam, is removed by the wire. This technique is disclosed in EP 481746. Surfactants may be of any suitable type, such as anionic, cationic, non-ionic and amphoteric surfactants. Additionally, wet-strengtheners, binders, starch-based binders, polyvinyl alcohol, latex creping chemicals etc. may be used.

Based on the above it can be seen that there exists a need to provide improved methods for the manufacture of structures based on peat moss, and new products comprising peat moss.

### SUMMARY

The present invention is based on studies relating to foam-laid methods and peat moss in order to produce porous composite structures based on peat moss. With said method composite structures may be obtained for various applications, for example such structures, which comply with present requirements of construction boards, acoustic boards etc.

The invention provides simple, continuous, flexible, economic and effective means for producing porous structures, such as boards, sheets, felts and blankets comprising peat moss.

The invention is directed to a method for the manufacture of composite structures comprising peat moss, wherein the method is a continuous foam-laid method, said method comprising the steps of:
- forming at least one foamed dispersion by dispersing fibers comprising 0.1 - 10% by weight of peat moss in a foamable liquid consisting of water, 0.005
- 10% by weight of one or more binders and 0.005 - 5% by weight of at least one foaming agent selected from anionic surfactants and polyvinyl alcohols,

- conveying the foamed dispersion or foamed dispersions to a foraminous support and draining liquid through the foraminous support to form a sheet or web, and
- drying the sheet or web.

The invention is further directed to a composite structure comprising one or more layers and consisting of 25 - 99 % by weight of peat moss, 0.001 - 0.1 % by weight of at least one foaming agent selected from anionic surfactants and polyvinyl alcohols, 0.01 - 5 % by weight of one or more binders, and it has a density of 10-250 kg/m³. Said composite structure is obtainable by the above method.

The invention is also directed to the use the composite structures comprising peat moss in liquid and oil absorption, in construction industry for insulation, as ballistic products, decorative products, in excavation, acoustics, agriculture, horticulture and landscaping applications.

The characteristic features of the invention are presented in the appended claims.

### BRIEF DESCRIPTION OF THE DRAWINGS

**Figure 1** illustrates graphically the fire resistance of the composite structures comprising peat moss according to the invention.
**Figure 2** illustrates graphically the sound absorption coefficients of structures comprising CTMP, peat and CTMP:peat as function of frequency (Hz), with 30 mm air gap behind the samples.
**Figure 3** illustrates graphically sound absorption coefficients of structures comprising CTMP, peat, CTMP:peat, and commercial reference product as function of frequency (Hz), without air gap behind the samples.
**Figure 4** illustrates graphically the sound insulation (dB) of structures comprising CTMP, peat and CTMP:peat as function of frequency (Hz).

### DEFINITIONS

Unless otherwise specified, the terms, which are used in the specification and claims, have the meanings commonly used in the field of forestry and pulp and paper industry. Specifically, the following terms have the meanings indicated below.

The term "peat moss" is understood here to mean the following components sedge peat, sphagnum peat, hare's tail cotton grass and any other naturally occurring peat and peat moss and any combinations thereof. The degree of decomposition may vary in said components.

"Foam-laying", also known as "foam-formation" refers here to any conventional monolayer and multi-layer foam-laid method used in the manufacture of non-woven products, tissue products and the like.

The term "foraminous support", also known as "foraminated support" refers here to a wire or the like.

The term "foaming agent" refers here to any surface active agent, foam producing agent, surfactant and combinations thereof, including tensides etc.

### DETAILED DESCRIPTION OF THE INVENTION

Peat moss is a slowly renewable material widely occurring typically in the northern countries. Peat moss may comprise sedge peat, sphagnum peat, hare's tail cotton grass any other naturally occurring peat and peat moss and any combinations thereof. Peat moss may comprise grades having different degree of decomposition. Peat moss is often regarded as very dusty, dirty and highly inhomogeneous material, and it requires several processing steps before it can be used in products requiring higher hygiene levels.

The conventional aqueous formation methods used in the manufacture of products comprising peat moss typically yield compact structures and high amounts of water and energy are consumed in the processes. Alternatively, products comprising peat moss are manufactured using polymeric binders and heat.

It was surprisingly found that highly porous, flexible composite structures comprising peat moss can be obtained utilizing a foam-laid method, in an effective, continuous and economic way. The composite structures may be designed and manufactured according to varying needs and specifications, with respect to the composition, thickness and porosity of the desired product.

The method for the manufacture of composite structures comprising peat moss, relates to the steps of:
- forming at least one foamed dispersion by dispersing fibers comprising peat moss in a foamable liquid comprising water and at least one foaming agent,
- conveying the foamed dispersion or dispersions to a foraminous support and draining liquid trough the foraminous support to form a sheet, and
- drying the sheet.

The present method for the manufacture of composite structures comprising peat moss, wherein the method is a continuous foam-laid method comprises the steps of:
- forming at least one foamed dispersion by dispersing fibers comprising 0.1 - 10% by weight of peat moss in a foamable liquid consisting of water, 0.005
- 10% by weight of one or more binders and 0.005 - 5% by weight of at least one foaming agent selected from anionic surfactants and polyvinyl alcohols,
- conveying the foamed dispersion or foamed dispersions to a foraminous support and draining liquid through the foraminous support to form a sheet or web, and
- drying the sheet or web.

Optionally at least one additional foamed dispersion is formed of fibrous material selected from natural fibers and synthetic fibers by dispersing said fibrous material in a foamable liquid comprising water and at least one foaming agent.

Optionally said foamed dispersions are conveyed to the foraminous support as individual layers.

The foraminous support is suitable a wire.

The draining is suitably carried out with the aid of vacuum, using vacuum pumps, or by gravitational filtration.

Drying of the formed web or sheet is suitably carried out for example by heating with means conventionally used in the manufacture of non-woven, paper and tissue products.

In the method the foamed dispersion (or dispersions) is formed of 0.1 - 10% by weight, preferably 0.5 - 8 % by weight, particularly preferably 1 - 5% by weight of peat moss, of 0.005 - 5% by weight, preferably 0.01 - 2% by weight, particularly preferably 0.01 - 1% by weight of at least one foaming agent, water and optional binders, optional fibrous materials and optional additives.

The amount of optional fibrous material may range from 0.1 to 10 % by weight, preferably 0.5 - 8 % by weight, particularly preferably 1 - 5% by weight.

The optional additional foamed dispersion (or dispersions) formed of fibrous material contain no peat moss. Said optional additional foamed dispersion (or dispersions) is formed of 0.1 - 10 % by weight, preferably 0.5 - 8 % by weight, particularly preferably 1 - 5 % by weight of at least one fibrous material selected from natural fibers and synthetic fibers, of 0.005 - 1 % by weight, preferably 0.01 - 0.05 % by weight, particularly preferably 0.01 - 0.03 % by weight of at least one foaming agent, water and optional binders and optional additives. The additional foamed dispersion (s) is conveyed individually on the support, whereby a product comprising at least two individual fiber layers is obtained.

The foamed dispersion comprises from 55 to 75 % by volume, preferably from 60 to 70 % by volume of air. Air refers here to all gases having more than 50 % by volume of nitrogen content, which includes atmospheric air or gases derived from atmospheric air.

The peat moss is selected from sedge peat, sphagnum peat, hare's tail cotton grass and any other naturally occurring peat/peat moss and any combinations thereof. The peat moss may optionally be subjected to one or more pretreatment steps selected from cutting, milling, screening, sieving, grinding and classification. Suitably fractions having average particle size in the range of 0.001 -50 mm are used.

The foaming agent may act as a surface active agent, which enables the formation of the foam and additionally it may act as a binder in the formed composite structure. The foaming agents include anionic, cationic, non-ionic and amphoteric surface active agents and surfactants, proteins, and any combination thereof, including polyvinyl alcohol and foamable starches. Said foaming agent is selected from anionic surface active agents, and polyvinyl alcohols and.

At least one additional binder is used in an amount of 0.005 - 10 % by weight, preferably 0.05 - 0.5 % by weight, particularly preferably 1 - 2 % by weight. Suitably the binder is selected from polyvinyl alcohols, polyvinyl acetate dispersions, ethyl vinyl alcohol dispersions, polyurethane dispersions, acrylic latexes, styrene butadiene dispersions, binders based on finely refined cellulose or cellulose derivatives, biopolymers such as binders based on starch derivatives, natural gum latexes, alginates, guar gum, hemicellulose derivatives, chitin, chitosan, pectin, agar, xanthan, amylose, amylopectin, alternan, gellan, mutan, dextran, pullulan, fructan, locust bean gum, carrageenan, glycogen, glycosaminoglycans, murein, bacterial capsular polysaccharides, and the like.

The optional fibrous material may comprise natural fibers and synthetic fibers. Natural fibers may be selected from chemical pulp, such as sulphate and sulphite pulp, organosolv pulp; recycled fibers; and/or mechanical pulp including e.g. refiner mechanical pulp (RMP), pressurized refiner mechanical pulp (PRMP), pretreatment refiner chemical alkaline peroxide mechanical pulp (P-RC APMP), thermomechanical pulp (TMP), thermomechanical chemical pulp (TMCP), high-temperature TMP (HT-TMP) RTS-TMP, alkaline peroxide pulp (APP), alkaline peroxide mechanical pulp (APMP), alkaline peroxide thermomechanical pulp (APTMP), Thermopulp, groundwood pulp (GW), stone groundwood pulp (SGW), pressure groundwood pulp (PGW), super pressure groundwood pulp (PGW-S), thermo groundwood pulp (TGW), thermo stone groundwood pulp (TSGW), chemimechanical pulp (CMP), chemirefinermechanical pulp (CRMP), chemithermomechanical pulp (CTMP), high-temperature CTMP (HT-CTMP), sulphite-modified thermomechanical pulp (SMTMP), reject CTMP (CTMPR), groundwood CTMP (G-CTMP), semichemical pulp (SC), neutral sulphite semi chemical pulp (NSSC), high-yield sulphite pulp (HYS), biomechanical pulp (BRMP), pulps produced according to the OPCO process, explosion pulping process, Bi-Vis process, dilution water sulfonation process (DWS), sulfonated long fibers process (SLF), chemically treated long fibers process (CTLF), long fiber CMP process (LFCMP), Kraft wood pulp, and modifications and combinations thereof. The pulp may be a bleached or non-bleached pulp. The pulp may originate from hardwood or softwood, including birch, beech, aspen such as European aspen, alder, eucalyptus, maple, acacia, mixed tropical hardwood, pine such as loblolly pine, fir, hemlock, larch, spruce such as Black spruce or Norway spruce, and mixtures thereof. Suitably CTMP pulp is used.

Also non-wood plant raw material, such as seed hair fibers, leaf fibers, bast fibers, plant fibers can be provided from e.g. straws of grain crops, wheat straw, reed canary grass, reeds, flax, hemp, kenaf, jute, ramie, seed, sisal, abaca, coir, bamboo, bagasse, cotton kapok, milkweed, pineapple, cotton, rice, reed, esparto grass, *Phalaris arundinacea,* or combinations thereof.

The synthetic fibers may comprise fibers of polyester, polyethylene, polypropylene, polylactide, rayon, lyocell, nylon, glass, polyacetate, aramide, carbon and any combinations thereof.

Additionally, optional additives may be used. Said additives may comprise wetting agents, wet-strengtheners, coloring agents, fire protection agents (e.g. borates, phosphates, magnesium trihydrate), nutrients, fertilizers, seeds, softening agents, inorganic fillers and any combinations thereof.

In the foam-laid method any equipment and apparatus used in foam-formation processes in the tissue paper and non-woven manufacture can be utilized here, such as suggested for example in GB 1397378, EP 481746 and US 3716449. Products comprising one or more foam-deposited layers may be obtained.

Optionally the dried product (such as sheet, felt, board, blanket etc) is coated or laminated on one side or on both sides with at least one layer comprising at least one polymer.

The applying of the polymer may be carried out by coating using spray coating, extrusion coating, curtain coating or foam coating.

In spray coating dispersions comprising polyvinyl alcohols (PVA), polyvinyl acetate dispersions, ethyl vinyl alcohol dispersions, polyurethane dispersions, acrylic latexes, styrene butadiene dispersions, starch based binders, finely refined cellulose or cellulose derivative based binders, biopolymers such as starch derivative based binders, natural gum latexes, alginates, guar gum, hemicellulose derivatives etc. are suitably used, in extrusion coating suitably polyethylene, polypropylene, polyamides, biopolymers such as polylactide, cellulose acetate, cellulose acetate butyrate, polyhydroxyalkanoate may be used.

The obtained product i.e. composite structure comprising peat moss may have a thickness of 1 - 200 mm, preferably from 1 to 100 mm. Also, a flexible product, such as a blanket or felt can be manufactured which may be winded on rolls, and it has a thickness of 0.5 to 5 cm, preferably from 1 to 2 cm.

The obtained product consists of from 25 to 99 % by weight of peat moss, preferably from 40 to 98 % by weight, particularly preferably from 55 to 98 % by weigh.

The composite structure comprising peat moss comprises from 0.001 to 0.1% by weight of at least one foaming agent.

The composite structure comprising peat moss additionally comprises from 0.01 to 5 % by weight of at least one binder.

The composite structure comprising peat moss may additionally comprise from 0.1 to 74 % by weight of synthetic fibers.

The composite structure comprising peat moss may additionally comprise from 0.1 to 74 % by weight of natural fibers.

The composite structure comprising peat moss is porous, with other words air containing light weight material with density of 10-250 kg/m³.

The properties of peat moss differ significantly from other cellulose based fibrous materials and thus also the properties of the obtained products are different.

Peat has naturally low pH (around 4-5) and acidity, which provides good adhesion with polymeric materials. Dry peat is hydrophobic and thus it does not absorb water, which is desired particularly in composite structures. Peat (peat fibers) absorb readily odors, oil, grease, gasoline and volatile organic compounds (VOCs). Peat provides the products (composites) soft feeling, "natural touch" and warmth

The composite structure comprising peat moss may be cut or formed to a board, blanket, felt, element or sheet or flexible felt. Said product may be from very stiff to very flexible depending on the end use. The manufacturing process may be adjusted accordingly.

If desired, products and elements may be formed of said structures, suitably polymeric binders are added to provide the desired rigidity when heat formed.

The composite structure comprising peat moss is used as absorbent board, sheet or blanket in liquid and oil absorption, absorbent felt in coffins, as an thermal insulation board/sheet or earth/ground frost insulation board/sheet, construction sheet, as decorative board/sheet in interior design applications, as geotextil or sheet in excavation, as acoustic board or sound absorption sheet, and in horticulture, landscaping and forestry applications, such as board, blanket, element or sheet for cultivation of plants, soil construction sheet (root support), biodegradable shelter, decoration item, flower holder, as packaging material such as thermo box, casing, shield material for packaging, absorption material in packaging, as moulded/shaped sheet, body works with thermosetting resins etc.

When compared to glass fiber insulation boards, to flax based insulation materials and cellulose based insulation materials the composite structure comprising peat moss is less expensive and simpler to manufacture, and it is based on natural materials. Boards and sheet complying with the present requirements in the construction industry can be met, and products according to the needs of end producers can be manufactured.

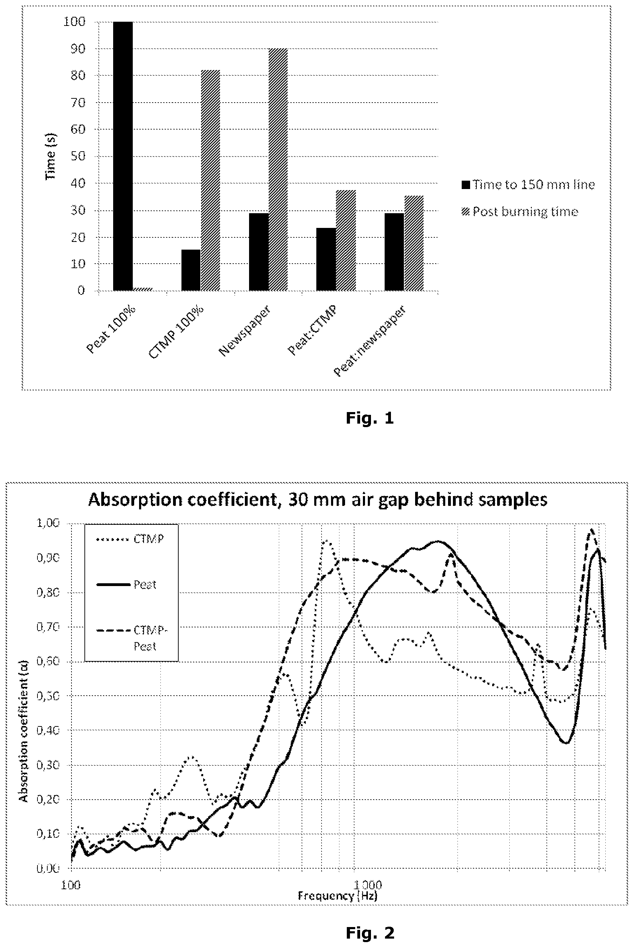

Surprisingly the fire resistance of the foam-laid composite structures comprising peat moss is very good. The products were tested according to standard EN 13501-1:2007, "Fire classification of construction products and building elements -Part 1: classification using test data from reaction to fire tests" with classes A2, B, C and D. In Example 1 boards were made of peat (100%), CTMP (100%), newspaper (100%), blend of peat and CTMP (50%, 50%) and blend of peat and newspaper (50%, 50%), without any fire retardants, and it was shown that the product containing peat moss may comply even with class B requirements. The results are illustrated in Figure 1. Thus, the invention provides fire resistant products suitable as constructions boards etc. If desired, additional fire retardants may be added to the products.

Also, particularly good thermal insulation materials, boards and the like can be manufactured having at least as good insulation properties as commercial flax based products.

The acoustic properties of the products comprising peat moss, such as sound absorption, particularly at low frequencies, are surprisingly good and thus the products may also be used in acoustic applications, for example in working machines where low frequencies are problematic.

The products comprising peat moss absorb readily very high amounts of oil, and they can be suitably used for collecting oil spills and the like.

The invention provides a continuous, effective and economic method for the manufacture of highly porous structures comprising peat moss, where smaller amounts of water are required in the processing, thus providing economical and environmental benefits. Completely bio-degradable products can be achieved if desired. The properties of the products can be tailored by adjusting the starting materials and process.

In horticultural use the structures may additionally comprise nutrients and fertilizers etc. according to the requirements of plants which are grown on the structure, as well as seeds if desired.

Biodegradable composite structures can be obtained based on natural peat moss, having excellent properties. Tailored products can be manufactured according to end user's need, the amounts of components, surfactants and binders may be varied resulting in different properties in the products. For example, when PVA is used, a soft windable felt is obtained. Also. highly porous products may be obtained.

If desired, the product may be coated on one side or on both sides with a polymeric layer, or the product may comprise a layer containing other fibrous material such as Kraft wood pulp on one side etc. Coating prevents dusting of the product.

When a multilayer foam-laid method is used, the layer purity is surprisingly high and a product with clearly separated layers is obtained. No adhesives are required between the layers.

### EXAMPLES

The following examples are illustrative of embodiments of the present invention, as described above, and they are not meant to limit the invention in any way.

### EXAMPLE 1

### Manufacture of composite structures

Foam-laid products were manufactured as follows. Partly sorted milled peat containing also hare's tail cotton grass was mixed with CTMP (chemi-thermo-mechanical pulp) or with disintegrated newspaper with a prefabricated foam. The foam was made from water containing a surfactant or polyvinyl alcohol. The foamed dispersion was conveyed to a wire, drained with suction and dried at 60 °C temperature. 0.5-10 cm thick porous sheets were obtained. The thinner sheets were flexible and windable. Boards made of peat (100%), CTMP (100%), newspaper (100%), blend of peat and CTMP (50%, 50%) and blend of peat and newspaper (50%, 50%), without any fire retardants were obtained.

### Recipe 1A

Partly sorted milled peat containing hare's tail cotton grass was mixed with prefabricated foam having air content of 66 % by volume. Said foam was made by mixing of water containing 1 % by weight of polyvinyl alcohol. The flexible and windable peat sheet was made using a foam hand sheet mold.

### Recipe 1B

Partly sorted milled peat containing hare's tail cotton grass was mixed with prefabricated foam having air content of 66 % by volume. Said foam was made by mixing of water containing 0.02 % by weight of anionic surfactant (sodium dodecyl sulphate). As a binder finely refined cellulose was added to the foamed dispersion in an amount of 10 % of peat dry weight. The rigid peat sheet was made using a foam hand sheet mold.

### Recipe 1C

Partly sorted milled peat containing hare's tail cotton grass and CTPM (spruce) or disintegrated newspaper (50%, 50%) was mixed with prefabricated foam having air content of 66 % by volume. Said foam was made by mixing from water containing 0.02 % by weight of anionic surfactant. The rigid peat-pulp sheet was made using a foam hand sheet mold.

### Fire Resistance

The obtained sheets/boards were tested for fire resistance according to standard EN ISO 11925-2:2010 - "Reaction to fire tests - Ignitability of products subjected to direct impingement of flame - Part 2: Single-flame source test" (ISO 11925-2:2010). Test conditions were 22°C, 30% RH, air flow during the test was 0.69 - 0.73 m/s. the ignition of the sample was done with 20 mm long propane gas flame in 45° angle for 15 s. Sample size was 250 mm x 90 mm with a thickness of 20 mm. Two parallel samples were tested in each test. Results are presented in following table 1.

**Table 1**

| SAMPLE | FIRE CATCH¹⁾ | FLAMES²⁾ | POST BURNING | COMMENTS |
|---|---|---|---|---|
| peat | yes | no | < 1 s | 5) |
| | yes | no | < 1 s | |
| CTMP | yes | 16 s | 1 min 22 s | 6) |
| | yes | 15 s | extinguished | |
| recycled paper | yes | 28 s³⁾ | over 1.5 min | 7) |
| | yes | 30 s³⁾ | over 1.5 min | |
| peat:CTMP | yes | 24 s | 38 s⁴⁾ | 8) |
| | yes | 23 s | 37 s⁴⁾ | |
| peat: recycled | yes | 28 s | 30 s⁴⁾ | 9) |
| paper | yes | 31 s | 41 s⁴⁾ | |

| | | | | |
|---|---|---|---|---|
| 1): Did the sample catch fire, flaming burn over 3 s. 2): Flames in 150 mm line. 3) Test was made in dark, but still it was difficult to see when the peak of the flame reached 150 mm line. 4): Flames were burned out oneself when they reached the metallic samples holder frame. 5): Both samples were left glowing, glow proceeded slowly, samples extinguished after 4.5 min. 6): Very strong flaming burn, followed by glowing. The other sample was extinguished due to strong burn after flames were at 150 mm line. 7): Due to strong flames both samples were extinguished after 1.5 min burn. 8): Weak flame, slowly proceeding glowing, samples extinguished after 2 min. 9): Weak flame, slowly proceeding glowing, samples extinguished after 2 min. | | | | |

Fire resistance is presented also in Figure 1 where the time of flame to reach 150 mm line and post burning time are presented for each sample. Accordingly, structures comprising peat fulfill the requirements of classes D or C and the best even class B.

### Thermal resistance

The obtained boards were tested for thermal resistance according to standard SFS-EN 12667 - "Thermal performance of building materials and products. Determination of thermal resistance by means of guarded hot plate and heat flow meter methods. Products of high and medium thermal resistance." The measurements were made at 10°C for the foam formed sheets of 400 x 400mm size, thickness 10 - 40 mm. Results are provided in table 2 below. As a reference, the thermal resistance in commercial flax insulation materials ranges in the area of 0.0350 - 0.0430 W/mK and in glass wool around 0.031 W/mK (density 60-80 kg/m³). Results of structures comprising peat are comparable with flax insulation materials.

**Table 2**

| Sample | Thickness d (mm) | Dry density P ¹⁾ (kg/m³) | Average Temperature T (°C) | Temperature difference ΔT (K) | Temperature flow density q (W/m²) | Thermal Resistance Λ₁₀ W/(mK) |
|---|---|---|---|---|---|---|
| CTMP | 35.6 | 23.2 | 10.35 | 18.60 | 21.36 | 0.0409 |
| peat | 60.0 | 67.2 | 10.34 | 18.63 | 11.13 | 0.0358 |
| peat | 35.2 | 24.9 | 10.36 | 18.55 | 22.56 | 0.0433 |
| peat: rc paper²⁾ | 60.0 | 43.2 | 10.36 | 18.55 | 12.37 | 0.0400 |
| peat: CTMP | 19.4 | 40.0 | 10.36 | 18.46 | 33.95 | 0.0357 |

| | | | | | | |
|---|---|---|---|---|---|---|
| 1): Sample thickness is dependent on sample preparation; there may be variation in different parts of sample. 2): Peat: recycled paper. | | | | | | |

### Air permeability

The obtained boards were tested for air permeability according to standard EN 29053. The measurement was carried out using three different pressure differences over the analyzed sample. Air permeability was calculated as an average of three results. Sample size was 200 × 200 mm and samples were placed one on another to reach the necessary sample thickness for the measurement.

Air permeability results were mostly higher than the ones for glass wool and flax insulation materials. The results are presented in following table 3.

**Table 3**

| Sample | Thickness of test Piece (mm) | Dry density¹⁾ P (kg/m³) | Air permeability L (W3/msPa) |
|---|---|---|---|
| CTMP | 45.0 | 18.3 | 880 x 10⁻⁶ |
| peat | 50.0 | 126.0 | 1063 x 10⁻⁶ |
| peat | 40.0 | 22.2 | 1180 x 10⁻⁶ |
| peat:rc paper | 50.0 | 81.5 | 1235 × 10⁻⁶ |
| peat:CTMP | 20.0 | 63.1 | 92 × 10⁻⁶ |

| | | | |
|---|---|---|---|
| 1): Sample thickness is dependent on sample preparation; there may be variation in different parts of sample. | | | |

### Sound absorption

Sound absorption (dB) and sound absorption coefficient (α) were measured for 30 mm and 100 mm round shaped foam formed samples with the thickness of 20 mm. Absorption coefficient and impedance were measured using two channel method with Brüel & Kjaer 4206 impedance pipe and pipes with two diameters. This method is based on standard ISO 10534-2.

Absorption coefficients of structures comprising CTMP, with thickness of 6.5 mm, peat with thickness of 11 mm and CTMP:peat (50%:50%) with thickness of 9.5 mm are presented in Figure 2 as function of frequency (Hz), with 30 mm air gap behind the samples.

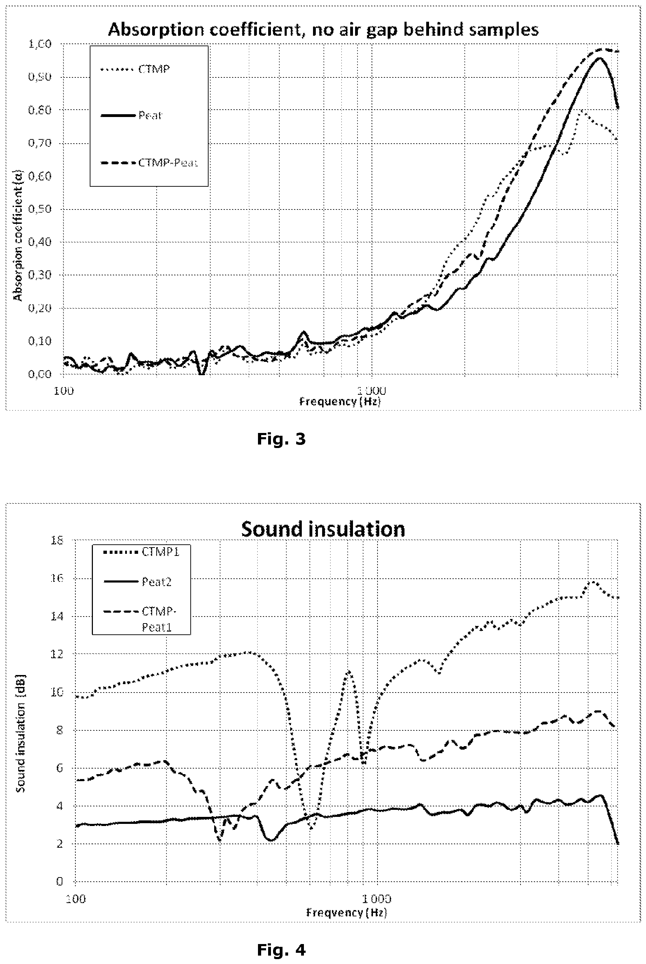

In Figure 3 absorption coefficients of structures comprising CTMP, with thickness of 6.5 mm, peat with thickness of 11 mm, CTMP:peat (50%:50%) with thickness of 9.5 mm, and commercial reference product (dust-free, unbreakable pressed felt having density of 1500 g/m²) are presented as function of frequency (Hz), without air gap behind the samples.

It can be seen that the absorption coefficient of CTMP:peat structure is comparable with the commercial reference. The sound absorption is moderate and it is related to sample air permeability and density, which both were low in these samples. Thicker samples with densities more than 20 mm yield higher sound absorption. Densities and air permeabilities of products can be adjusted during the manufacturing process.

Figure 4 presents sound insulation (dB) as a function of frequency (Hz), measured for the same samples as in Figure 3. Peat provides low sound insulation and it may be used for tailoring properties of materials having high sound insulation.

### Oil absorption

Oil absorption was tested with the peat structure having thickness of 2 cm. Rapeseed oil was added drop wise on the surface of a 1g sample of the dry peat sheet until oil was leaking out from the sample. The amount of absorbed oil was measured. 1 g sample of peat sheet was able to absorb 14 g of rapeseed oil, even without changing its shape.

## Claims

1. A method for the manufacture of composite structures comprising peat moss, **characterized in that** the method is a continuous foam-laid method comprising the steps of:
- forming at least one foamed dispersion by dispersing fibers comprising 0.1 - 10% by weight of peat moss in a foamable liquid consisting of water, 0.005
- 10% by weight of one or more binders and 0.005 - 5% by weight of at least one foaming agent selected from anionic surfactants and polyvinyl alcohols,
- conveying the foamed dispersion or foamed dispersions to a foraminous support and draining liquid through the foraminous support to form a sheet or web, and
- drying the sheet or web.

2. The method according to claim 1, **characterized in that** the foamed dispersion comprises 0.5 - 8% by weight of peat moss and 0.01 - 2 % by weight of at least one foaming agent.

3. The method according to claim 1 or 2, **characterized in that** the fibers comprising peat moss comprise additionally one or more of natural fibers and synthetic fibers.

4. The method according to any one of claims 1 - 3, **characterized in that** the foamed dispersion comprises additionally coloring agents, nutrients, fertilizers, seeds, wetting agents, wet strengtheners and softening agents.

5. The method according to any one of claims 1 - 4, **characterized in that** at least one additional foamed dispersion is formed by dispersing fibrous material comprising natural fibers or synthetic fibers or combinations thereof in a foamable liquid consisting of water and at least one foaming agent.

6. The method according to any one of claims 1 - 5, **characterized in that** the foamed dispersions are conveyed to the support as individual layers.

7. The method according to any one of claims 1 - 6, **characterized in that** after drying the sheet or web is coated on one side or on both sides with a polymer.

8. A composite structure, **characterized in that** it is obtained by the method of any one of claims 1 - 7 and it comprises one or more layers, it consists of 25 - 99 % by weight of peat moss, 0.001 - 0.1 % by weight of at least one foaming agent selected from anionic surfactants and polyvinyl alcohols, 0.01 - 5% by weight of one or more binders, and optionally one or more of natural fibers and synthetic fibers, coloring agents, nutrients, fertilizers, seeds, wetting agents, softening agents, wet-strengtheners, and it has a density of 10-250 kg/m³.

9. The composite structure according to claim 8, **characterized in that** it comprises at least two individual fiber layers.

10. The composite structure according to claim 8 or 9, **characterized in that** it comprises a polymeric layer on one side or both sides.

11. Use of the composite structure according to any one of claims 8 - 10 as absorbent board, sheet or blanket in liquid and oil absorption, as absorbent felt in coffins, as an thermal insulation board/sheet or earth/ground frost insulation board/sheet, as construction sheet, as decorative board/sheet in interior design applications, as geotextil or sheet in excavation, as acoustic board or sound absorption sheet, in horticulture, landscaping and forestry applications as board, blanket, element or sheet for cultivation of plants, soil construction sheet or root support, biodegradable shelter, decoration item, flower holder, as packaging material, shield material for packaging, absorption material in packaging, as moulded/shaped sheet or element.

## Patentansprüche

1. Verfahren zur Herstellung von Verbundstrukturen, die Torfmoos umfassen, **dadurch gekennzeichnet, dass** das Verfahren ein kontinuierliches Schaumlegeverfahren ist, das die Schritte umfasst:
- Ausbilden mindestens einer geschäumten Dispersion durch Dispergieren von Fasern, die 0,1 - 10 Gew.% Torfmoos umfassen, in einer schäumbaren Flüssigkeit bestehend aus Wasser, 0,005 - 10 Gew.% eines oder mehrerer Bindemittel und 0,005 - 5 Gew.% mindestens eines Schäumungsmittels, das aus anionischen oberflächenaktiven Stoffen und Polyvinylalkoholen ausgewählt ist,
- Fördern der geschäumten Dispersion oder geschäumten Dispersionen zu einer porösen Auflage und Abführen von Flüssigkeit durch die poröse Auflage zur Ausbildung eines Blattes oder einer Bahn, und
- Trocknen des Blattes oder der Bahn.

2. Verfahren nach Anspruch 1, **dadurch gekennzeichnet, dass** die geschäumte Dispersion 0,5 - 8 Gew.% Torfmoos und 0,01 - 2 Gew.% mindestens eines Schäumungsmittels umfasst.

3. Verfahren nach Anspruch 1 oder 2, **dadurch gekennzeichnet, dass** die Torfmoos umfassenden Fasern außerdem natürliche Fasern und/oder synthetische Fasern umfassen.

4. Verfahren nach einem der Ansprüche 1 bis 3, **dadurch gekennzeichnet, dass** die geschäumte Dispersion außerdem Farbstoffe, Nährstoffe, Düngemittel, Saatgut, Benetzungsmittel, Nassfestmittel und Enthärtungsmittel umfasst.

5. Verfahren nach einem der Ansprüche 1 bis 4, **dadurch gekennzeichnet, dass** mindestens eine zusätzliche geschäumte Dispersion ausgebildet wird, indem Fasermaterial, das natürliche Fasern oder synthetische Fasern oder Kombinationen derselben umfasst, in einer schäumbaren Flüssigkeit, die aus Wasser und mindestens einem Schäumungsmittel besteht, dispergiert wird.

6. Verfahren nach einem der Ansprüche 1 bis 5, **dadurch gekennzeichnet, dass** die geschäumten Dispersionen als einzelne Lagen zur Auflage gefördert werden.

7. Verfahren nach einem der Ansprüche 1 bis 6, **dadurch gekennzeichnet, dass** das Blatt oder die Bahn nach dem Trocknen einseitig oder beidseitig mit einem Polymer beschichtet wird.

8. Verbundstruktur, **dadurch gekennzeichnet, dass** sie durch das Verfahren nach einem der Ansprüche 1 bis 7 erhalten wird und eine oder mehrere Schichten umfasst, aus 25 - 99 Gew.% Torfmoos, 0,001 - 0,1 Gew.% mindestens eines Schäumungsmittels, das aus anionischen oberflächenaktiven Mitteln und Polyvinylalkoholen ausgewählt ist, 0,01 - 5 Gew.% eines oder mehrerer Bindemittel, und optional natürlichen Fasern und/oder synthetischen Fasern, Farbstoffen, Nährstoffen, Düngemitteln, Saatgut, Benetzungsmitteln, Enthärtungsmitteln, Nassfestmitteln besteht und eine Dichte von 10-250 kg/m³ aufweist.

9. Verbundstruktur nach Anspruch 8, **dadurch gekennzeichnet, dass** sie mindestens zwei einzelne Faserschichten umfasst.

10. Verbundstruktur nach Anspruch 8 oder 9, **dadurch gekennzeichnet, dass** sie auf einer Seite oder auf beiden Seiten eine Polymerschicht umfasst.

11. Verwendung der Verbundstruktur nach einem der Ansprüche 8 bis 10 als absorbierende Platte, Bahn oder Matte bei der Flüssigkeits- und Ölabsorption, als saugfähigen Filz in Särgen, als Wärmedämmplatte/-bahn oder Erd-/Bodenfrostdämmplatte/-bahn, als Baumaterialbahn, als Dekorplatte/-flächenelement in Innenarchitekturanwendungen, als Geotextil oder Bahnmaterial beim Erdaushub, als Akustikplatte oder Schallschluckplatte, in gartenbaulichen, landschaftsbaulichen und forstwirtschaftlichen Anwendungen als Platte, Matte, Element oder Bahn für den Anbau von Pflanzen, Bodenaufbaumatte oder Wurzelauflage, biologisch abbaubares Schutzelement, Dekorartikel, Blumenhalter, als Verpackungsmaterial, Abschirmmaterial für Verpackungen, Absorptionsmaterial in Verpackungen, als geformte Bahn oder geformtes Element.

## Revendications

1. Procédé de fabrication de structures composites comprenant de la tourbe de sphaigne, **caractérisé en ce que** le procédé est un procédé de formation par mousse en continu qui comprend les étapes consistant à
- former au moins une dispersion moussée en dispersant des fibres comprenant 0,1 à 10 % en poids de tourbe de sphaigne dans un liquide moussable composé d'eau, de 0,005 à 10 % en poids d'un ou plusieurs liants et de 0,005 à 5 % en poids d'au moins un agent moussant choisi parmi les agents tensioactifs anioniques et les alcools polyvinyliques,
- acheminer la dispersion moussée ou les dispersions moussées vers un support poreux et évacuer du liquide à travers le support poreux pour former une feuille ou une bande, et
- faire sécher la feuille ou la bande.

2. Procédé selon la revendication 1, **caractérisé en ce que** la dispersion moussée comprend 0,5 à 8 % en poids de tourbe de sphaigne et 0,01 à 2 % en poids d'au moins un agent moussant.

3. Procédé selon la revendication 1 ou 2, **caractérisé en ce que** les fibres comprenant de la tourbe de sphaigne comprennent également des fibres naturelles et/ou fibres synthétiques.

4. Procédé selon l'une des revendications 1 à 3, **caractérisé en ce que** la dispersion moussée comprend également des colorants, nutriments, engrais, semences, agents mouillants, agents de résistance à l'état humide et adoucisseurs.

5. Procédé selon l'une des revendications 1 à 4, **caractérisé en ce qu'**au moins une dispersion moussée supplémentaire est formée par la dispersion d'un matériau fibreux comprenant des fibres naturelles ou fibres synthétiques ou combinaisons de celles-ci dans un liquide moussable composé d'eau et d'au moins un agent moussant.

6. Procédé selon l'une des revendications 1 à 5, **caractérisé en ce que** les dispersions moussées sont acheminées vers le support en tant que couches individuelles.

7. Procédé selon l'une des revendications 1 à 6, **caractérisé en ce qu'**une face ou les deux faces de la feuille ou de la bande est/sont revêtue(s) d'un polymère.

8. Structure composite, **caractérisée en ce qu'**elle est obtenue par le procédé selon l'une des revendications 1 à 7 et comprend une ou plusieurs couches, est composée de 25 à 99 % en poids de tourbe de sphaigne, de 0,001 à 0,1 % en poids d'au moins un agent moussant choisi parmi les agents tensioactifs anioniques et les alcools polyvinyliques, de 0,01 à 5 % en poids d'un ou plusieurs liants, et, en option, de fibres naturelles et/ou fibres synthétiques, colorants, nutriments, engrais, semences, agents mouillants, adoucisseurs, agents de résistance à l'état humide, et présente une densité de 10-250 kg/m³.

9. Structure composite selon la revendication 8, **caractérisée en ce qu'**elle comprend au moins deux couches fibreuses individuelles.

10. Structure composite selon la revendication 8 ou 9, **caractérisée en ce qu'**elle comprend une couche polymérique sur une face ou sur les deux faces.

11. Utilisation de la structure composite selon l'une des revendications 8 à 10 comme panneau, feuille ou matelas absorbant(e) pour l'absorption des liquides et huiles, comme feutre absorbant dans les cercueils, comme panneau/feuille d'isolation thermique ou panneau/feuille d'isolation contre le gel des sols/terres, comme membrane de construction, comme panneau décoratif / feuille décorative dans les applications de décoration intérieure, comme géotextile ou feuille dans l'excavation, comme panneau acoustique ou feuille d'insonorisation, dans les applications d'horticulture, d'aménagement paysager et de sylviculture, comme panneau, matelas, élément ou feuille pour la culture végétale, feuille de construction de sol ou support de racines, abri biodégradable, article décoratif, porte-fleurs, comme matériau d'emballage, matériau de protection pour les emballages, matériau absorbant dans les emballages, comme feuille ou élément moulé(e)/façonné(e).
